# EUROPÄISCHE PATENTSCHRIFT

(11) **EP 0 554 765 B1**
(45) Veröffentlichungstag und Bekanntmachung des Hinweises auf die Patenterteilung: **16.04.1997**
(21) Anmeldenummer: 93101155.5
(22) Anmeldetag: 27.01.1993
(51) Int. Cl.: C07C 13/20, C07C 5/11

(54) **Verfahren zur Herstellung von Cyclohexen durch partielle Hydrierung von Benzol**
Process for preparing cyclohexene by partial hydrogenation of benzene
Procédé pour la préparation de cyclohexène par hydrogénation partielle de benzène

(30) Priorität: 05.02.1992 DE 4203220
(43) Veröffentlichungstag der Anmeldung: 11.08.1993
(73) Patentinhaber: BASF Aktiengesellschaft, 67063 Ludwigshafen (DE)
(72) Erfinder: Fischer, Rolf, Dr., W-6900 Heidelberg (DE); Dostalek, Roman, Dr., W-6725 Roemersberg (DE); Marosi, Laszlo, Dr., W-6700 Ludwigshafen (DE)

(56) Entgegenhaltungen:
- US-A- 3 912 787
- US-A- 4 197 415
- US-A- 4 575 572
- US-A- 4 734 536

## Beschreibung

Die Erfindung betrifft ein Verfahren zur Herstellung von Cyclohexen durch partielle Hydrierung von Benzol mit Wasserstoff in Gegenwart von Rutheniumkatalysatoren und Wasser.

Aus der US-Patentschrift 4 197 415 ist bekannt, daß man cyclische Olefine, z.B. Cyclohexen durch partielle Hydrierung von aromatischen Kohlenwasserstoffen wie Benzol in Gegenwart von Rutheniumkatalysatoren erhält. Die dort verwendeten Rutheniumkatalysatoren sind Tränkkatalysatoren auf Trägern wie Mordenit und enthalten als Promotoren Phosphate von Metallen der 2. bis 8. Nebengruppe des periodischen Systems. Mit einem Ruthenium/Nickelkatalysator auf einem zeolithischen Träger werden hierbei Cyclohexenausbeuten von 14 mol-% mit einer Selektivität von 29 % erhalten. Diese Werte sind verbesserungsbedürftig.

Nach einem anderen in der US-Patentschrift 3 912 787 beschriebenen Verfahren erhält man Cyclohexen durch Hydrierung von Benzol in Gegenwart von Rutheniumkatalysatoren, die Mangan, Kobalt oder Nickel als Promotoren enthalten. Die Promotoren können hierbei getrennt von Ruthenium in die Reaktionszone eingeführt werden. Hierbei erzielt man Ausbeuten bis zu 20 mol-% Cyclohexen mit einer Selektivität von 34 % bei einer Verweilzeit von 62 min. Um eine Übertragung in den technischen Maßstab zu ermöglichen, sind die erzielten Ergebnisse verbesserungsbedürftig.

Nach einem anderen in der EP-A 55 495 beschriebenen Verfahren wird die Herstellung von Cycloalkenen, z.B. Cyclohexen durch partielle Gasphasenhydrierung von aromatischen Kohlenwasserstoffen wie Benzol in Gegenwart eines Rutheniumkatalysators unter Mitverwendung von Wasserdampf beschrieben. Neben Ruthenium kann der Katalysator ein oder mehrere Metalle oder Metallverbindugnen aus der Reihe Eisen, Chrom, Germanium, Blei, Zink, Nickel und vorzugsweise Natrium enthalten. Die Hydrierung in der Gasphase hat den Nachteil, daß große Reaktionsräume erforderlich sind und somit die Raumzeitausbeute sinkt.

Es war deshalb die technische Aufgabe gestellt, ein Verfahren zur Herstellung von Cyclohexen durch partielle Hydrierung von Benzol zur Verfügung zu stellen, bei dem man hohe Umsätze mit hohen Selektivitäten an Cyclohexen erzielt, kurze Verweilzeiten einhält und eine hohe Raumzeitausbeute mit einem weniger anfälligen Katalysator erreicht.

Diese Aufgabe wird gelöst in einem Verfahren zur Herstellung von Cyclohexen durch partielle Hydrierung von Benzol mit Wasserstoff in Gegenwart von Wasser und Ruthenium/Nickelkatalysatoren bei erhöhter Temperatur unter erhöhtem Druck in flüssiger Phase, dadurch gekennzeichnet, daß man Legierungen von Ruthenium mit Nickel als Katalysatoren verwendet, mit der Maßgabe, daß man keine Katalysator-Träger verwendet.

Das neue Verfahren hat den Vorteil, daß es mit verbesserten Raumzeitausbeuten verläuft und höhere Umsätze und erhöhte Selektivitäten zu Cyclohexen erzielt werden. Weiter hat das neue Verfahren den Vorteil, daß kurze Verweilzeiten ermöglicht werden und der Katalysator eine verbesserte Lebensdauer aufweist.

Erfindungsgemäß geht man von Benzol aus und hydriert dieses mit Wasserstoff partiell zu Cyclohexen. Vorteilhaft wird die Umsetzung bei einer Temperatur von 20 bis 300°C durchgeführt. Besonders bewährt hat sich eine Temperatur von 70 bis 250°C, insbesondere 100 bis 200°C. Hierbei hält man vorteilhaft einen Wasserstoffpartialdruck von 1 bis 200 bar, vorzugsweise 10 bis 100 bar ein. Die Hydrierung wird in flüssiger Phase durchgeführt. D.h. die Temperatur- und Druckbedingungen sind so aufeinander abzustimmen, daß die Einhaltung einer flüssigen Phase gewährleistet wird.

Ein wesentliches Merkmal der Erfindung ist es, daß die Umsetzung in Gegenwart von Legierungen von Ruthenium mit Nickel als Katalysatoren durchgeführt wird. Geeignete Ruthenium-Nickellegierungen haben einen Nickelgehalt z.B. von 0,01 bis 50 Gew.-%, bezogen auf die Summe von Ruthenium und Nickel. Vorteilhaft beträgt der Nickelgehalt von 0,1 bis 35 Gew.-%, insbesondere hat sich ein Nickelgehalt von 1 bis 30 Gew.-% bewährt.

Geeignete Ruthenium/Nickel-Legierungen sind beispielsweise erhältlich, indem man Rutheniumverbindungen, die in Rutheniumoxid überführbar sind, wie Ruthenium(III)-Chlorid oder Ruthenium(III)-nitrat, Ruthenium(III)-chloro-pentamminchlorid zusammen mit Nickelverbindugnen, die in Nickeloxid überführbar sind, wie Nickel(II)-Chlorid, Nickel(II)-Sulfat, Nickel(II)-Nitrat oder Nickelacetat in Form einer wäßrigen Lösung, z.B. 1 bis 8 gew.-%ig unter kräftigem Rühren mit Alkalilauge versetzt, die vorteilhaft einen Gehalt von 10 bis 40 Gew.-% hat. Hierbei hält man zweckmäßig eine Temperatur von 20-100°C ein. Der erhaltene Niederschlag wird anschließend noch 1 bis 3 Stunden bei einer Temperatur von 60 bis 100°C, insbesondere 70 bis 90°C weitergerührt und anschließend der Niederschlag absitzen gelassen. Der erhaltene Niederschlag wird von der Lösung abdekantiert oder abfiltriert und dann mit leicht alkalischem Wasser, vorteilhaft mit einem pH-Wert von 10 bis 11, frei von Salzen gewaschen. Der so erhaltene Niederschlag wird in einem Hochdruckgefäß mit wäßriger Alkalilauge mit einem pH-Wert von 10 bis 11 aufgeschlämmt. Anschließend wird unter einem Wasserstoffpartialdruck von 1 bis 200 bar vorteilhaft 10 bis 100 bar, insbesondere 20 bis 80 bar und einer Temperatur von 50 bis 300°C, vorteilhaft 100 bis 270°C, insbesondere 120 bis 250°C unter Rühren für einen Zeitraum von 4 bis 8 Stunden, insbesondere 5 bis 7 Stunden unter Rühren aktiviert. Das so erhaltene Katalysatorpulver wird von der Natronlauge abgetrennt, z.B. durch Dekantieren und anschließend unter Schutzgasatmosphäre wie Stickstoff oder Argon mit Wasser neutral gewaschen und dann unter vermindertem Druck bei erhöhter Temperatur z.B. 40 bis 70°C getrocknet.

Vorteilhaft sind Ruthenium/Nickel-Legierungen, deren Kristallgitterkonstanten für die a-Achse von 0,2610 bis 0,2705 nm und für die c-Achse von 0,4200 bis 0,4280 nm beträgt. Diese Werte belegen, daß es sich nicht um Mischungen aus elementarem Ruthenium mit Nickel handelt, sondern um eine echte Legierung.

Die Röntgenbeugungsaufnahmen werden mit einem SIEMENS-Diffraktometer D-500 erstellt, das mit einem Sekundärmonochromator und Szintillationszähler ausgestattet ist. Es wird Kupfer-Kα-Strahlung (40 kV, 30 mA) angewendet. Zur Bestimmung der genauen Linienlagen wird den Proben Zinkoxid als innerer Standard beigemischt und die Gitterkonstanten a₀ und c₀ der Katalysatorprobe aus der Lage der Reflexe (100) und (102) berechnet.

Vorteilhaft wendet man, bezogen auf das eingesetzte Benzol von 0,001 bis 50 Gew.-% Rutheniumnickellegierung an. Besonders bewährt haben sich Mengen von 0,05 bis 10 Gew.-%, insbesondere 0,1 bis 2 Gew.-%.

Die Umsetzung wird in Gegenwart von Wasser durchgeführt. Hierbei soll die Wassermenge, bezogen auf die Gesamtflüssigkeitsmenge, vorzugsweise von 5 bis 90 Gew.-% betragen. Vorteilhaft enthält die wäßrige Phase ein oder mehrere gelöste Kationen von Übergangsmetallen der 2. bis 8. Gruppe des periodischen Systems wie Chrom, Mangan, Eisen, Kobalt, Zink oder Ammonium in Form ihrer Chloride, Nitrate, Acetate, Phosphate oder Sulfate. Besonders bevorzugt sind Kobalt und Zinksulfat. Hiervon insbesondere Zinksulfat. Der pH-Wert dieser Salzlösung ist vorteilhaft neutral oder schwach sauer z.B. ein pH-Wert von pH 3 bis pH 7. Die Menge an Metallsalz, bezogen auf die wäßrige Phase, beträgt vorteilhaft von 0,1 Gew.-% bis zur Sättigungskonzentration.

Ferner hat es sich bewährt, dem Reaktionsgemisch mindestens eines der Metalloxide, ausgewählt aus der Reihe Aluminiumoxid, Siliciumdioxid, Zirkoniumdioxid, Titandioxid, Hafniumdioxid, Chromtrioxid und Zinkoxid zuzusetzen. Vorzugsweise beträgt die Menge an zugesetztem Metalloxid von 5 · 10⁻⁴ bis 20 Gew.-%, bezogen auf die eingesetzte Wassermenge.

Die Hydrierung kann sowohl diskontinuierlich als auch kontinuierlich, z.B. in Suspension oder mit einem fest angeordeten Katalysator durchgeführt werden.

Das nach dem Verfahren der Erfindung erhältliche Cyclohexen eignet sich zur Herstellung von Cyclohexanol, einem wichtigen Ausgangsstoff für die Herstellung von Faservorprodukten.

Das Verfahren nach der Erfindung sei in folgenden Beispielen veranschaulicht:

### Beispiel 1

15 g Ruthenium(III)-Chlorid x 3H₂O und 5,6 g Nickel(II)-Chlorid x 6H₂O werden in 700 ml Wasser gelöst und unter kräftigem Rühren zügig mit 100 ml einer 20%igen Natronlauge versetzt. Den erhaltenen Niederschlag läßt man noch etwa 2 Stunden bei 80°C weiterrühren und anschließend innerhalb von 10 Stunden absitzen. Danach wird die über dem Niederschlag stehende Lösung abdekantiert und der Niederschlag mit insgesamt 1000 ml leicht alkalischem Wasser (pH-Wert 11) neutral gewaschen. Nach Überführung in einen 11-Rührautoklav mit Teflonauskleidung wird zusammen mit 500 ml einer stark verdünnten Natronlauge (pH-Wert 10-11) bei 50 bar Wasserstoffdruck und 170°C sechs Stunden lang gerührt. Das entstandene Katalysatorpulver wird in einer Argonatmosphäre mit Wasser neutral gewaschen und anschließend im Vakuum bei 60°C getrocknet. Man erhält 7,5 g Katalysator-Legierung mit einem Nickel-Gehalt von 20 Gew.-%.

0,3 g dieser Katalysatorlegierung, 9,6 g ZnSO₄ x 7 H₂O, 1,2 g ZrO₂, 95 ml Wasser und 45 ml Benzol werden in einem Autoklav mit 300 ml Innenvolumen unter kräftigem Rühren auf 150°C erhitzt. Nach Erreichen dieser Temperatur wird bis zu einem Druck von 50 bar Wasserstoff aufgepreßt und eine vorbestimmte Zeit lang weitergerührt. Danach wird die organische Phase abgetrennt und gaschromatographisch analysiert. Die Ergebnisse sind aus Tabelle 1 zu entnehmen.

### Beispiel 2

Die Benzolhydrierung wurde wie in Beispiel 1 mit einem Katalysator durchgeführt, dessen Nickel-Gehalt nach der Herstellungsvorschrift in Beispiel 1, jedoch durch Einsatz einer entsprechend größeren Menge an NiCl₂ x 6 H₂O auf 24,3 Gew.-% eingestellt wurde.

### Beispiel 3

Die Benzolhydrierung wurde wie in Beispiel 2, jedoch ohne ZnSO₄-Zusatz durchgeführt.

### Beispiel 4

Die Benzolhydrierung wurde wie in Beispiel 2, jedoch mit 9,6 g CoSO₄ statt 9,6 g ZnSO₄ durchgeführt.

### Beispiel 5

Die Benzolhydrierung wurde wie in Beispiel 1 mit einem Katalysator, der 17 Gew.-% Nickel enthiel bei einer Reaktionstemperatur von 170°C durchgeführt.

### Beispiel 6

Die Benzolhydrierung wurde wie in Beispiel 1 mit einem Katalysator, der 16 Gew.-% Nickel enthielt durchgeführt.

### Beispiel 7

Die Benzolhydrierung wurde wie in Beispiel 1 mit einem Katalysator, der 15 Gew.-% Nickel enthielt durchgeführt.

### Beispiel 8 und 9

Die Benzolhydrierung wurde wie in Beispiel 5, jedoch mit 9,6 g ZnCl₂, bzw. mit 9,6 g CoSO₄ statt ZnSO₄ durchgeführt.

### Beispiele 10 und 11 (Vergleichsbeispiele)

Die Benzolhydrierung wurde wie in Beispiel 1 mit Gemischen aus fein verteiltem Ruthenium- und Nickelpulver als Hydrierkatalysatoren durchgeführt. Die Ergebnisse in Tabelle 1 zeigen, daß mit Legierungen dieser beiden Elemente deutlich höhere Cyclohexen-Ausbeuten und -Selektivitäten zu erreichen sind.

Die erzielten Ergenisse sind in folgender Tabelle aufgeführt.

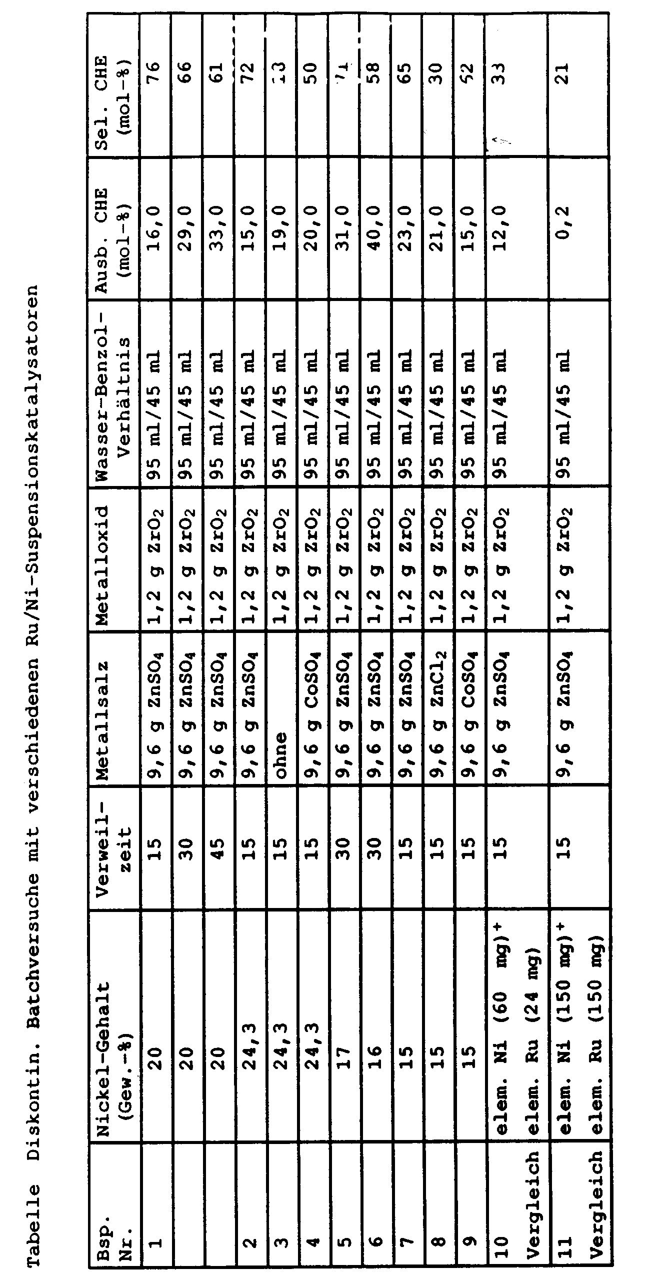

## Patentansprüche

1. Verfahren zur Herstellung von Cyclohexen durch partielle Hydrierung von Benzol mit Wasserstoff in Gegenwart von Wasser und Rutheniumkatalysatoren, die mit Nickel modifiziert sind, bei erhöhter Temperatur in flüssiger Phase, dadurch gekennzeichnet, daß man Legierungen von Ruthenium und Nickel als Katalysatoren verwendet, mit der Maßgabe, daß man keine Katalysator-Träger verwendet.

2. Verfahren nach Anspruch 1, dadurch gekennzeichnet, daß man Ruthenium/Nickel-Legierungen mit einem Nickelgehalt von 1 bis 30 Gew.-% verwendet.

3. Verfahren nach den Ansprüchen 1 und 2, dadurch gekennzeichnet, daß die Ruthenium/Nickel-Legierungen für die a-Achse eine Kristallgitterkonstante zwischen 0,2610 und 0,2705 nm und für die c-Achse zwischen 0,4200 und 0,4280 nm aufweisen.

4. Verfahren nach den Ansprüchen 1 bis 3, dadurch gekennzeichnet, daß die Wassermenge, bezogen auf die Gesamtflüssigkeitsmenge von 5 bis 90 Gew.-% beträgt.

5. Verfahren nach den Ansprüchen 1 bis 4, dadurch gekennzeichnet, daß die Wasserphase neutral oder schwach sauer ist.

6. Verfahren nach den Ansprüchen 1 bis 5, dadurch gekennzeichnet, daß die wäßrige Phase ein oder mehrere gelöste Kationen von Übergangsmetallen der 2. bis 8. Gruppe des Periodensystems als Promotoren enthält.

7. Verfahren nach den Ansprüchen 1 bis 6, dadurch gekennzeichnet, daß man Zinksulfat und/oder Kobaltsulfat als Promotoren verwendet.

8. Verfahren nach den Ansprüchen 1 bis 7, dadurch gekennzeichnet, daß man mindestens eines der Metalloxide Aluminiumoxid, Siliziumdioxid, Zirkoniumdioxid, Titandioxid, Hafniumdioxid, Chromtrioxid und Zinkoxid zusetzt.

9. Verfahren nach den Ansprüchen 1 bis 8, dadurch gekennzeichnet, daß man 5·10⁻⁴ bis 20 Gew.-% Metalloxide, bezogen auf die Wassermenge zusetzt.

10. Verfahren nach den Ansprüchen 1 bis 9, dadurch gekennzeichnet, daß man die Umsetzung bei einer Temperatur von 100 bis 200°C durchführt.

## Claims

1. A process for the preparation of cyclohexene by partial hydrogenation of benzene with hydrogen in the presence of water and a ruthenium catalyst which is modified with nickel, at elevated temperatures in the liquid phase, wherein an alloy of ruthenium and nickel is used as the catalyst, with the proviso that no catalyst carrier is employed.

2. A process as claimed in claim 1, wherein a ruthenium/nickel alloy having a nickel content of from 1 to 30% by weight is used.

3. A process as claimed in claims 1 and 2, wherein the ruthenium/nickel alloy has a crystal lattice constant a of from 0.2610 to 0.2705 nm and a crystal lattice constant c of from 0.4200 to 0.4280 nm.

4. A process as claimed in any of claims 1 to 3, wherein the amount of water is from 5 to 90% by weight, based on the total amount of liquid.

5. A process as claimed in any of claims 1 to 4, wherein the aqueous phase is neutral or slightly acidic.

6. A process as claimed in any of claims 1 to 5, wherein the aqueous phase contains one or more dissolved cations of transition metals of the 2nd to 8th group of the Periodic Table as promoters.

7. A process as claimed in any of claims 1 to 6, wherein zinc sulfate or cobalt sulfate is used as the promoter.

8. A process as claimed in any of claims 1 to 7, wherein at least one of the metal oxides alumina, silica, zirconium dioxide, titanium dioxide, hafnium dioxide, chromium trioxide and zinc oxide is added.

9. A process as claimed in any of claims 1 to 8, wherein from 5·10⁻⁴ to 20% by weight, based on the amount of water, of metal oxides are added.

10. A process as claimed in any of claims 1 to 9, wherein the reaction is carried out at from 100 to 200°C.

## Revendications

1. Procédé pour la préparation de cyclohexène par hydrogénation partielle de benzène avec de l'hydrogène en présence d'eau et de catalyseurs au ruthénium, qui sont modifiés avec du nickel, à température élevée en phase liquide, caractérisé en ce qu'on utilise des alliages de ruthénium et de nickel à titre de catalyseurs, sous condition qu'on n'utilise pas de supports de catalyseurs.

2. Procédé selon la revendication 1, caractérisé en ce qu'on utilise des alliages de ruthénium/nickel avec une teneur en nickel de 1 à 30 % en poids.

3. Procédé selon les revendications 1 et 2, caractérisé en ce que les alliages de ruthénium/nickel présentent une constante de réseau cristallin pour l'axe a entre 0,2610 et 0,2705 nm et pour l'axe c entre 0,4200 et 0,4280 nm.

4. Procédé selon les revendications 1 à 3, caractérisé en ce que la quantité d'eau s'élève à 5 à 90 % en poids par rapport à la quantité totale de liquide.

5. Procédé selon les revendications 1 à 4, caractérisé en ce que la phase aqueuse est neutre ou faiblement acide.

6. Procédé selon les revendications 1 à 5, caractérisé en ce que la phase aqueuse contient un ou plusieurs cations solubilisés de métaux de transition du 2^{e} au 8^{e} groupe du système périodique à titre de promoteurs.

7. Procédé selon les revendications 1 à 6, caractérisé en ce qu'on utilise du sulfate de zinc et/ou du sulfate de cobalt à titre de promoteurs.

8. Procédé selon les revendications 1 à 7, caractérisé en ce qu'on ajoute au moins un des oxydes métalliques : oxyde d'aluminium, dioxyde de silicium, dioxyde de zirconium, dioxyde de titane, dioxyde d'hafnium, trioxyde de chrome et oxyde de zinc.

9. Procédé selon les revendications 1 à 8, caractérisé en ce qu'on ajoute 5 · 10⁻⁴ à 20 % en poids d'oxydes métalliques, par rapport à la quantité d'eau.

10. Procédé selon les revendications 1 à 9, caractérisé en ce qu'on effectue la conversion à une température de 100 à 200 °C.
